# EUROPEAN PATENT APPLICATION

(11) **EP 3 918 925 A1**
(43) Date of publication of application: **08.12.2021**
(21) Application number: 20747871.0
(22) Date of filing: 31.01.2020
(51) Int. Cl.: A23L 21/25, C13K 3/00, C12P 19/02, A23L 33/15, A23L 33/16, A23L 33/21

(54) **ARTIFICIAL HONEY COMPOSITION AND PRODUCTION PROCESS**

(30) Priority: 01.02.2019 BR 102019002150
(71) Applicant: Nidus-Tec-Desenvolvimento de Produtos e Processos Tecnologicos Ltda, 13457-050 Santa Bárbara d'Oeste (BR)
(72) Inventor: RISCHBIETER, Ivo, 89015-405 BLUMENAU (BR); BIONDO, Ronaldo, 13468-150 Americana (BR)
(74) Representative: dompatent von Kreisler Selting Werner - Partnerschaft von Patent- und Rechtsanwälten mbB
(86) International application number: PCT/BR2020/050022
(87) International publication number: WO 2020/154788

(57) **Abstract**

The present invention relates to a production process, composition and use of an artificial honey based on the use of inverted sugar, preferably using the enzyme invertase from yeast, with the addition of nutritive components such that the final syrup has a formulation similar to that of natural honey and can have improved pharmaceutical functions and biological functions, being healthier. The artificial honey of the present invention relates to a product of strictly plant origin, which is produced without the use of animal work or biological materials, and can be commercialized in various sectors, including the vegan sector. More specifically, the artificial honey of the present invention is produced from inverted sucrose, preferably by means of the enzymatic catalysis of VHP/ VVHP/demerara brown sugars rich in minerals, vitamins and natural antioxidants of great pharmacological interest and various types of substances of interest. The artificial honey of the present invention has no chemical substances that are harmful to human health (e.g. pigments, preservatives or other chemical additives) added to it or produced in it, but can be flavored and/or aromatized with artificial honey flavoring, flavoring identical to natural honey and, optionally, natural honey flavoring, and may also contain fiber, vitamin supplements, minerals, vitamins, amino acids, natural extracts, being optionally directly consumed or used for manufacturing other food products.

## Description

### FIELD OF THE INVENTION

This invention relates to the production of an artificial honey product encompassing chemical or enzymatic inversion of sugars, with the optional addition of nutritive and functional components for producing a formula similar to or healthier than natural honey without using bees or any other animal.

The artificial honey of this invention refers to a plant-based product, produced without using the labor or any biological material derived from animals. More specifically, it is produced based on inverted sucrose, preferably by means of an enzymatic catalysis of VHP/VVHP/demerara dark sugars rich in minerals, vitamins, and natural antioxidants with wide nutritional and pharmacological appeal. It does not contain or produce any chemical substances harmful to humans (e.g. furfural) or added starch; however, it can be flavored and/or aromatized with a honey aroma identical to the natural flavor, an artificial one, and optionally a natural aroma added with fibers, natural extracts, vitamins, minerals, and amino acids. The honey product of this invention may be consumed either directly or in other food, pharmaceutical, nutraceutical, or cosmetic products.

### BACKGROUND OF THE INVENTION

Bee honey has been used by the human kind both as food and as medicine since times immemorial. As a matter of fact, ancestral records indicate that raw honey is the oldest sweetener and it has been used all over the world for many millions of years: ***[***Crane E: History of honey. In Honey, A comprehensive Survey. Edited by Crane E. London: William Heinemann; 1975:439-488***].***

Bee honey is a sweet and tasteful liquid food with a high nutritional value and several health benefits ***[***Bogdanov S, Jurendic T, Sieber R, Gallmann P: Honey for Nutrition and Health: A Review. J Am Co/1 Nutr 2008, 27(6):677-689***;*** Ajibola A, Idowu GO, Amballi AA, Oyefuga OH, Iquot IS: Improvement of some haematological parameters in albino rats with pure natural honey. J Biai Sei Res 2007, 2:67-69***].***

It is produced by bees from the nectar of flowers and plant exudates. In addition, it is widely consumed, and its use goes beyond the barriers of culture and ethnicity.

Honey use is even defended and employed by all religious and cultural beliefs. It is even a food mentioned in all religious books and accepted by all generations, traditions, and civilizations, old and modern. For instance, the religion of Islam recommends using honey as both food and medicine and even includes a full chapter in the Holy Quran called Surah al-Nahl, which means "the bee honey chapter" ***[***An-Nahl (The Bee) 16, 1-128: The Holy Qur'an, English translation of the meanings and Commentary. The Presidency of Islamic Researches, IFTA, Cal/ and Guidance***.*** Al-Madinah Al- Munawarah: Kingdom of Saudi Arabia: King Fahd Holy Qur'an Printing Complex; 1990:730-773. 1410 A.H.5***].***

Besides, it is reported in the Holy Bible that John the Baptist survived on a diet including wild honey for a long period of time when he was in the desert, as well as that King Solomon said: "My son, eat honey, for it is good;" [Old Testament, proverb 24:13; Mathew 3, 4: The Holy Bible. Authorised King James Version. New York: Oxford University Press; 1972. 10***].***

Other groups of beliefs, traditions, and civilizations that attest to the popularity of honey include Buddhists, Jews, Hindus, Vedas, and others ***[***Jones R: Honey and healing through the ages. In Honey and healing. Edited by Munn P, Jones R. Cardiff: International Bee Research Association IBRA; 2001***;*** Crane E: History of honey. In Honey, A comprehensive Survey. Edited by Crane E. London: William Heinemann; 1975:439-488***].***

Currently, the world production of bee honey is around 1.2 million tons, less than 1% of the total production of sugar in the world ***[***Alvarez-Suarez JM et al. Contribution of honey in nutrition and human health: a review. Mediterr J Nutr Metab 2010, 3:15-23***].*** Honey consumption varies a lot from country to country. The largest exporters of honey, China and Argentina, have small consumptions of 0.1 to 0.2 per capita a year. Honey consumption is higher in developed countries, whose production not always meets their market demands.

In the European Union, which is at the same time a large importer and producer of honey, annual consumption per capital varies from medium (0.3 to 0.4 kg) in Italy, French, Great Britain, Denmark, and Portugal, to high (1.0 to 1.8 kg), in Germany, Austria, Switzerland, Portugal, Hungary, and Greece, whereas in countries like the USA, Canada, and Australia, the average consumption per capital is 0.6 to 0.8 kg/year ***[***Bogdanov S, Jurendic T, Sieber R et al. Honey for nutrition and health: a review. Am J Co/1 Nutr 2008, 27:677-6897***].***

The global market of bee honey is estimated at between 2.0 and 6.0 billion dollars ***[***Ajibola A, Idowu GO, Amballi AA, Oyefuga OH, Iquot IS: Improvement of some haematological parameters in albino rats with pure natural honey. J Biai Sei Res 2007, 2:67-69***].***

Honey has always been obtained by man in an extractive manner and, often, with harm to beehives. Over the centuries, man learned how to capture swarms and install them in "artificial hives".

By developing and enhancing the handling techniques, they managed to increase the production of honey and extract it without damaging the hive. With the "domestication" of bees for honey production, apiculture started. Currently, on top of honey, several products can be obtained, such as bee pollen, royal jelly, apitoxin, propolis, and wax. Also, queen bees and, in some cases, swarms and breeds are produced and traded.

Generally, honey is mostly made up (around 75%) of carbohydrates, notably simple sugars (glucose and fructose). Honey is also made up of water (around 20%), minerals (calcium, copper, iron, magnesium, phosphorus, potassium, and others), around half of the existing amino acids, organic acids (acetic acid, citric acid, and others), and complex-B vitamins, vitamin C, D, and E ***[***Ajibola, A, Chamunorwa, JP; Erlwanger, KH. Nutraceutical values of natural honey and its contribution to human health and wealth. Nutrition & Metabolism 2012, 9:61***].*** Natural honey also has elements in its composition such as hormones, pigments, amino acids, and other biologically active substances, as described in document CN108208266, titled "Formula of honey product", filed on 06/29/2018**,** which is being considered herein; therefore, not only is it a food with a high energy value, but it is also an important supplement to human nutrition.

More specifically, to manufacture natural honey, the bee secretes two enzymes, **invertase** and glucose oxidase, through glands existing on its head. Honey is formed from a reaction of those substances to the nectar (mainly sucrose) collected from flowers.

Each of these enzymes has a certain role in honey production. **Invertase** converts sucrose - type of sugar contained in nectar - into two other sugars: **glucose and fructose.** Glucose oxidase, in its turn, transforms a small quantity of glucose into gluconic acid, which makes honey acidic, protecting it from bacteria. These bacteria, if uncontrolled, would make honey ferment.

In addition, by shaking their wings, bees remove the water present in a large quantity in the nectar, thus dehydrating the honey, which helps prevent microorganisms from spreading.

However, it has been observed that the quality of the honey available on the world market is not constant, since it would require a strict quality control certifying where the bees collect pollen. This is not always possible. The quality of bee honey depends on its chemical composition and floral origin. The composition of the active components in plants depends on many factors, particularly biochemistry, chemotype, and weather conditions ***[***Alvarez-Suarez JM et al. Contribution of honey in nutrition and human health: a review. Mediterr J Nutr Metab 2010, 3:15-23***].*** For that reason, there is no standardization regarding the types and composition of honey products produced around the world, making it greatly difficult to establish a quality control over both their nutritional properties and their pharmacological and medical properties.

Honey, like any other natural food, may also be exposed to contamination by antibiotics, pesticides, heavy metals, and other toxic compounds ***[***Bogdanov S: Contaminants of bee products. Apidologie 2006, 38:1-18***].***

These hazardous substances may result from disease control in crops using agrochemicals, accidental exposure, ***environmental risks, air pollution, and hostile human practices [***Schneider A: Asian honey, banned in Europe, is flooding U.S. grocery shelves, Food Safety News. 7th edition. Seattle Washington: The Food Watchdog; 2011. Assessed from http://www.food safefynews.com/2011/08/honeylaundering/ on 04/14/12***;*** Bibi S, Husain SZ, Malik RN: Pollen analysis and heavy metals detection in honey samples from seven selected countries. Pak J Bot 2008, 40(2):507-516***].***

For example, it was reported by European health authorities that lead (Pb) had been found in honey bought from India in early 2010. The results showed, in addition to lead, at least two antibiotics in nearly 23.0% of all 362 test samples of the export honey product ***[***Schneider A: Asian honey, banned in Europe, is flooding U.S. grocery shelves, Food Safety News. 7th edition. Seattle Washington: The Food Watchdog; 2011. Assessed from http:llwww.foodsafetynews.com/2011/08/honeylaundering/ on 14/04/12***]**,* this being a concerning health issue for honey consumers.

Analyzing the same scenario through a different prism, the United Nations environment program has already warned about bee mortality especially in the North hemisphere. The main reason is believed to be the use of agrochemicals in crops, but they also mention viral epidemics and parasites that can be devastating for that insect, which is very fragile ***[***Ratnieks FLW, Carreck N. Clarity on honey bee collapse? Science, 2010, 327, 152-153***].***

According to several sources, such as the U.S. Colony Collapse Disorder Steering Committee, pollination is threatened and plant productivity is falling ***[***Ratnieks and Carreck, Clarity on Honey Bee Collapse? Science, 2010, 327: 152- 153***].*** The United Nations Environment Agency has issued a report that the world population of bees will continue to be in decline, unless man changes his way of handling the planet, causing great environmental and food security concerns.

From that perspective, the history of human life shows that men have been feeding from fruit, plants, and animal meat. However, during our evolutionary history, some groups began to oppose the consumption of animal products. A religious group established in the 6^{th} century BC created Orphism, which banished animal sacrifice and consumption.

Another example of a movement opposing the consumption of animal products was Greek mathematician Pythagoras, who believed in reincarnation and, for that reason, refrained from consuming meat. For a long time, the religious issue began to exert wide influence on the people's decision not to consume animal products ***[***Spencer, Colin. The Heretic's Feast: A History of Vegetarianism. Lebanon: UPNE, 1996. 402 p***].*** Only in the middle of the 19^{th} century did the first vegetarian organizations start to show up, the first of them in England, in 1847. Following that event, other countries also began to establish vegetarian organizations, like the United States, in 1850, and Germany, in 1867. For example, the International Vegetarian Union (IVU) was founded in 1908 ***[***Leitzmann, Claus. Vegetarian nutrition: past, present, future. American Journal Of Clinical Nutrition 2014, 100:496-502***].***

These days, the vegetarian movement has been growing all over the world. According to recent data, India has 40% of its population adopting vegetarianism ***[***Leitzmann, Claus. Vegetarian nutrition: past, present, future. American Journal Of Clinical Nutrition 2014, 100: 496-502***;*** Ruby M. Vegetarianism: A blossoming field of study. Appetite. Vancouver, 2012, 58: 141-150***].*** In developed countries, the percentage of vegetarians in the total population varies from 2 to 9%, and in the United States and the United Kingdom they comprise approximately 3% ***[***Vegetarian Journal. How many vegetarians are there? Baltimore, 2009***.*** The Vegetarian Resource Group; Food Standards Agency. Public Attitudes to Food. London. 91 p., 2009***],*** 8% in Canada and in Brazil ***[***Vancouver Human Society. Almost 12 million Canadians now vegetarian or trying to eat less meat. Available at: http://www.vancouverhumanesociety.bc, visited on: Nov. 20, 2018***;*** IBOPE. Dia Mundial do Vegetarianismo: 8% da população brasileira afirma ser adepta do estilo. Available at: http://www.ibope.com.br, visited on: Nov. 20, 2018***],*** 9% in Germany and Italy, and 2% in France, Spain, and Portugal ***[***EVU - European Vegetarian Union. V-label guide. Available at: <https:llwww.v-label.eu/v-label-guide>. Visited on: Nov. 20 2018***].***

In many countries, the segment of vegetarian consumers has been growing expressively: in Australia, it increased by 30% from 2012 to 2016, when the percentage of that population became 11% vegetarians ***[***Roy Morgan. The slow but steady rise of vegetarianism in Australia. Available at: <http://www.roymorgan.com>, visited on: Nov. 20 2018***].***

In the last decades of the 20^{th} century, vegetarian nutrition started to be scientifically assessed through research on the various diets adopted, seeking to understand their benefits for man. From that moment on, more and more studies demonstrate that a balanced vegetarian diet can be completely healthy, which draws the attention of individuals to that movement ***[***Leitzmann, Claus. Vegetarian nutrition: past, present, future. American Journal Of Clinical Nutrition 2014, 100: 496-502***].***

Nowadays, the vegetarian movement accounts for important segments of consumers, especially in the developed countries, and, for that reason, it has been the subject of study by researchers and organizations who characterize the new trends in consumption of food in the world ***[***Euromonitor International. Top 10 Global Consumer Trends for 2016. 45 p. 2016***].***

Several reasons lead people to become vegetarians, with the most common argument relating to the ethics issue of animal slaughter, a search for personal health, and support to food production systems that generate a reduced environmental impact ***[***Ruby M. Vegetarianism: A blossoming field of study. Appetite. Vancouver 2012, 58:141-150***].***

There are strict vegetarian people who will not consume any animal product. On the other hand, there are those who occasionally eat fish and still call themselves vegetarians ***[***Ruby M. Vegetarianism: A blossoming field of study. Appetite. Vancouver 2012, 58:141-150***].***

Generally, vegetarians are deemed to be individuals who do not consume any type of red meat, poultry, or fish. However, this type of diet has its sub-classifications, which define the degree of exclusion of each animal food, encompassing a wide spectrum of nutrition standards. According to Key et al. (2006), ***[***Key TJ, Appleby PN, Rosell MS. Health effects of vegetarian and vegan diets. Proceedings Of The Nutrition Society 2006, 65:35-41***],*** a vegetarian diet can be classified as follows:
- **Lacto-ovo-vegetarian:** does not consume any type of meat, but consumes eggs, milk, and their byproducts;
- **Lacto-vegetarian:** does not consume any type of meat or eggs, but consumes milk and dairy products;
- **Ovo-vegetarian:** does not consume any type of meat, milk, or dairy products, but consumes eggs;
- **Strict vegetarian:** does not consume any animal product whatsoever in their nutrition;
- **Semi-vegetarian:** consumes some types of meat (usually white meat) in a reduced quantity, as well as eggs, milk, and dairy products;
- **Vegan:** does not consume any type of meat or animal product in their nutrition, such as milk, eggs, and honey. Also, they do not use animal products or products manufactured in production processes that used animals, such as leather, suede, silk, skins, cosmetics tested on animals, etc.
-

The vegan food market is the most well-defined of all existing markets, with world certification standards and a better outlined field of study.

Vegan certification has international standards that are common to all countries having authorities that grant that seal. This standard was created by the European Vegetarian Union (EVU) and, later, adopted by other countries.

According to the EVU ***[***EVU - European Vegetarian Union. Definitions of "vegan" and "vegetarian" in accordance with the EU Food Information Regulation. Berlin. 5p. 2016***],*** the certification criteria for vegan foods are:
- They cannot contain any animal ingredient, like meats and substances coming from animal parts or secretions;
- They cannot contain animal additives, supplements, or enzymes;
- They cannot be processed with technology adjuvants that are of animal origin.

Besides, throughout product development and manufacturing, no animal may be used in any stage, including toxicity or experimentation tests. Certification is granted per product, rather than granted to a company as a whole. Thus, the maximum percentage of contamination of animal products in vegan products is 0.1% ***[***EVU - European Vegetarian Union. How many veggies are there? Available at: <http://www.euroveg.eu/lang/en/infolhowmany.php>. Visited on: Oct. 18, 2017***;*** EVU - European Vegetarian Union. V-label guide. Available at: <https://www.v-label.eu/v-label-guide>. Visited on: Nov. 02, 2017**].**

There is high trend in this segment looking for products that meet the vegan market. However, honey cannot be consumed by vegans, because it comes from bees.

In view of all such dilemmas, it would be interesting to develop a production process for different compositions than those of the artificial honey products available today. These artificial honey products use starch as a raw material, and their composition is not truly analogous to natural honey.

On the other hand, to produce honey without using bees, it is necessary to develop a production method that dispenses with animal use or labor in any form, which will allow this exceptional food to be supplied to all the population in the event of a future scenario where bee exploitation is restricted, whether this population consumes conventional honey or follows the vegan philosophy.

### PURPOSES OF THE INVENTION

The invention herein described has the following purposes:
- To supply artificial honey using a sustainable process, contributing toward the preservation of bees and, as a consequence, cooperating in the pollination process that is key in farming activities;
- To supply an artificial honey product containing fibers that allow for a slower digestion of carbohydrates, avoiding insulin peaks and improving the gut flora. It is worth lighting that natural honey does not contain fibers;
- To provide an artificial honey product free of microorganisms, that is, without a pathogenic risk;
- Finally, to propose an artificial honey product that conserves that pharmacological potential due to the use of brown sugars, such as VHP or Demerara sugars, which have pharmacologically active compounds in their natural composition.

### SUMMARY OF THE INVENTION

This invention provides, therefore, a solution for the problems found in the state of the art, providing a method for artificial honey production that obtains a formula very similar to natural honey, though from plant-based sucrose. That is, it does not use any material of animal origin and does not employ the labor of bees, who suffer with human activity and often with the environmental pressure of viruses and pathogens.

The first modality of this invention shows a composition with uniform quality, since it depends on a source of sucrose and is independent from flowers or outside interferences, as is the case with bee-based production. This provides consumers with assurance of a food complement with a controlled origin, a food product in accordance with the specific demands of each type of consumer.

The second modality of this invention shows a process of production of an artificial honey product, preferably from the raw material sucrose, that is, inverted sucrose, preferably using enzymes (not chemically) to make even more similar to natural honey. The process of this invention will not use undesirable added chemical substances, such as colorants and artificial pigments and others, and may be flavored preferably with a honey aroma identical to the natural flavor (vegan), artificial aroma, and, as an option, the natural aroma. The process may further include the optional addition of isolated elements of mineral or plant origin that are naturally found in bee honey, such as amino acids, whole proteins, vitamins, minerals, etc. Thus, a different formulation can be obtained when compared to the artificial honey products available in the state of the art and on the market.

The third and last modality of this invention consists in using the artificial honey either as a household food product and in the general industry, for instance, in byproducts such as cakes, sweets, dairy, cosmetics, jellies, sweets, food supplements, drugs, nutraceuticals, cereal bars, and others.

This invention, therefore, is based on sucrose produced on demand for the production of artificial honey. For that purpose, the artificial honey so produced may have as its preferred origin the VHP/VVHP/Demerara brown sugars, which are rich in minerals, vitamins, and natural antioxidants with wide pharmacological appeal.

However, production of the honey product in this invention may be based on different sources of carbohydrates, but preferably from VHP (Very High Polarization) and Demerara sugars inverted using the invertase enzyme, or non-animal origin, similar to the one used by bees.

In other words, to keep the characteristic of the artificial honey very close to natural honey, this invention uses the invertase enzyme itself, preferably through a fully natural process, biologically and in a GMO-free method (a technology that does not use genetically modified organisms) or using commercially available enzymes.

### DETAILED DESCRIPTION OF THE INVENTION

This invention therefore describes an ARTIFICIAL HONEY COMPOSITION AND PRODUCTION PROCESS based on inverted sugar, reaching a carbohydrate composition that is very close to that of natural bee honey, that is, glucose and fructose in similar quantities and reduced sucrose quantity.

In view of the possibility that other functional additives are used, the artificial honey product of this invention may have a lower glycemic index than the average/typical value in bee honey. To adjust the aroma of the product, aromas identical to the natural flavors are used, and no artificial colorant whatsoever is used.

For instance, using the VHP or Demerara sugar as a basis for manufacturing the artificial honey allows the product to have an important set of mineral salts, vitamins, and antioxidants coming from the sugarcane, as described in the following example:

The ARTIFICIAL HONEY COMPOSITION AND PRODUCTION PROCESS is conducted with 100 grams of VHP or Demerara sugar, and several minerals are present. It is worth highlighting here that the mineral composition directly depends on the type of sugarcane cultivar, the agricultural treatment, crop region, etc. However, several studies show that, the darker and, obviously, the less "treated" a sugar product is, the more minerals will be conserved in it, as shown by Silva (2017) ***[***Silva, AFS. Caracterização e determinação de minerais em amostras de açúcares brasileiros. Master's Thesis. ESALQ, USP, Piracicaba, 2017***],*** as shown in Table 1, below:

**Table 1:** concentration range determined for minerals analyzed by Inductively Coupled Plasma Atomic Emission Spectroscopy (ICP OES) in the various types of sugar:

| **Element** | **Refined (mg Kg⁻¹)** | **Coarse (mg Kg⁻¹)** | **Demerara (mg Kg⁻¹)** | **Brown (mg Kg⁻¹)** |
|---|---|---|---|---|
| **Mn** | 0.005 to 0.30 | 0.03 to 0.4 | 0.2 to 1.5 | 4.4 to 25.8 |
| **Mq** | 0.6 to 15.7 | 2.6 to 32.5 | 12 to 79 | 175.8 to 1065.5 |
| **K** | 0.45 to 69.5 | 4.7 to 81 | 14 to 49 | 439.8 to 2891.5 |
| **S** | 5 to 37 | 16 to 102 | 41.4 to 163.7 | 56.3 to 1154.5 |
| **P** | 0.1 to 9 | 1.5 to 9 | 4.5 to 20.2 | 30 to 338.1 |
| **Zn** | 0.07 to 0.5 | 0.07 to 1 | 0.09 to 0.36 | 1.05 to 5.6 |
| **Ca** | 4 to 101.5 | 106 to 1214 | 80.2 to 244.2 | 275 to 2346 |
| **Cu** | 0.005 to 0.230 | 0.01 to 0.30 | 0.06 to 0.2 | 0.35 to 1.7 |
| **Fe** | 1.7 to 17.5 | 0.8 to 9 | 3.3 to 57.8 | 223.5 to 298.2 |
| **Si** | 7.5 to 32 | 10 to 27 | 11.4 to 65.4 | 50 2808 |

As shown in **Table 1,** darker sugars have increasing mineral contents, going from refined sugar to brown sugar. Even when you compare Demerara sugar to Refined sugar, we observe a huge difference in the contents of all minerals shown in **Table 1.**

The more extensive the knowledge on the exact composition of each of the sugars that we can use as a basis for artificial honey, for instance, the higher the power to select the most adequate raw material for producing artificial honey product that is richer as food or exhibiting desired organoleptic, physical-chemical, and/or sensory properties. In this regard, choosing an optimal source of sucrose is essential and a technology advantage so that the artificial honey product of this invention will not only become more similar to natural honey, but will also exhibit improved properties when compared to natural honey.

To better illustrate the above-mentioned improvements, the artificial honey product of this invention may contain pharmacological compounds, for instance, those present in VHP and Demerara sugars, such as flavonoids and phenolic acids such as luteolin, apigenin, tricine, quercetin, kaempferol caffeic acid, apigenin, luteolin, tricine, chlorogenic acid, coumaric acid, and ferulic acid. These compounds are directly associated with several pharmacological activities such as antioxidant, anti-inflammatory, antimicrobial, and even anti-tumor properties, as shown in **Table 2: *[***Valli V, Gomez-Caravaca A.M.; D/ Nunzio M., Danesi F, Caboni MF, Bordoni A. Sugar cane and sugar beet molasses, antioxidant-rich alternatives to refined sugar. J. Agri Food Chem, 2012, 60, 12508-12515***;*** Alves V.G, Souza AG, Chiavelli LUR, Ruiz ALTG, Carvalho JE, Pomini AM, Silva CC. Phenolic compounds and anticancer activity of commercial sugarcane cultivated in Brazil. Na. Acad. Bras, Cienc. 2016, 88, 1201-1209***;*** Taylor R P. Discovery of bioactive natural products from sugarcane. Master of Science Thesis. School of Environmental Science and Management, Southern Cross University, Lismore NSW, Australia. 2018***;*** Almeida JMD. Flavonóides e ácidos cinâmicos de cana-de-açúcar (Saccharum officinarum L. - Poaceae) e seus produtos. Identificação e atividade antioxidante e antiproliferativa. Doctor's Thesis. University of São Paulo, 2006***].***

This invention therefore shows an artificial honey composition capable of keeping the pharmacological properties of brown sugars that are demonstrated to help maintain the muscle tone of the digestive tract wall; improve the health of the nervous system; strengthen the skin, nails, and hair; improve the functioning of the liver, speed up the healing of injuries, and prevent and treat anemia due to the iron present. These attributes, depending on the raw material and additives used, allow the artificial honey product of this invention to be compared to the most famous natural honey, *Manuka* honey, derived from the *Manuka* flower in New Zealand, regarded as one of the best in the world.

The honey product of this invention may exhibit important medical properties, depending on the raw material and additives used as similar to or better than those of the *Manuka* honey, since its composition includes different bioactive compounds derived, from instance, from the brown cane sugar, as can be observed in **Table 2.** In addition, the artificial honey product does not contain pesticides, which were detected in a *Manuka* honey study conducted by Moniruzzaman et al (2014) ***[***Moniruzzaman M, Chowdhury MAZ, Rahman MA, Sulaiman SA, Gan SH. Determination of mineral, trace element, and pesticide levels in honey samples originating from different regions of Malaysia compared to Manuka Honey. Biomed. Res. Int. 2014, ID 359890***].***

**Table 2: comparison between Manuka and the honey product of this invention, which has inverted VHP sugar, for instance, as its raw material.**

| **Product** | **Composition** | **Role** |
|---|---|---|
| **Manuka** | **Flavonoids:** quercetin, luteolin, kaempferol, pinocembrine, chrysin, galangin, pinobanksin | Antioxidant, anti-inflammatory |
| | **Phenolic:** caffeic acid, coumaric acid, gallic acid, hydroxybenzoic acid, syringic acid | antioxidant, antimicrobial |
| | **Peroxidases:** leptosin, methyl syringate | Peroxide/antimicrobial activity |
| | | |
| | **Carbohydrates:** 85 g / 100 g | |
| | **Fats:** 0 q | |
| | **Proteins:** 5 q | |
| | **Calories:** 300 Kcal | |
| **honey** / **inverted VHP** | **Flavonoids:** luteolin, apigenin, tricine, quercetin, kaempferol | antioxidant, anti-inflammatory, anti-tumor |
| | **Phenolic:** caffeic acid, apigenin, luteolin, tricine, chlorogenic acid, coumaric acid, ferulic acid | antioxidant, antimicrobial |
| | **Carbohydrates:** 80 g / 100 g | |
| | **Fats:** 0 g | |
| | **Proteins:** 0 g | |
| | **Calories:** 307 Kcal | |

Moreover, the process of this invention conducts a sucrose inversion elevating its sweetness from a level of 100 to 120, with a 20% increase, very close to the sweetening power of bee honey, to adjust to the demands of consumers and the industry, who also demand or will demand a real substitute for natural honey.

In the inversion proposed in this invention, for example, above 98%, sucrose is almost completely converted into equal parts of glucose and fructose, without significantly losing its nutritional characteristics as to vitamins, antioxidants, and minerals present prior to inversion. Because it is a biological catalyst, the invertase enzyme (preferably free of genetically modified organisms - GMO) or a non-GMO-free invertase enzyme, unlike chemical inversion, does not form toxic compounds in the inversion process, such as furfural.

With a very low content of residual sucrose, an optional addition of functional fibers may allow the honey product of this invention to exhibit a glycemic index deemed low (<55). This makes it more adequate for diets seeking to avoid illnesses, such as diabetes and obesity problems.

To illustrate it better, the characteristics of this artificial honey product are shown below, made from Demerara sugar, for example, as per this invention:
- Conventional or organic artificial honey, depending on the origin of the Demerara sugar used;
- Produced from inverted Demerara sugar (> 98%), largely converted into glucose and fructose, with a sweetness index very close to bee honey;
- With additional fructose or functional fibers, exhibiting a low glycemic index (<55);
- Artificial honey with an antioxidant power due to the polyphenolic compounds of dark sugars. These compounds are preserved in the enzymatic inversion process;
- GMO-free or non-GMO-free enzymes;
- 100% natural process, without artificial conservatives or any chemical additive;
- Due to the on-site production of the invertase enzyme, competitive production cost;
- Some pharmacological potential in view of the substances preserved in dark sugars like Demerara;
- Vitamins, minerals, amino acids, and fibers may be added that are usually found in natural honey, for instance, complex-B vitamins, such as B1, B2, and B6, soluble and/or insoluble fibers, and essential or non-essential amino acids, antioxidants and minerals, for instance, iron, zinc, and potassium chelates and others, but which can also be preserved in dark sugars depending on the inversion process.
-

**Artificial Honey Production Process,** according to this invention, in one of the forms of artificial honey production, the method starts with dilution of solid sucrose, present in the selected source of carbohydrate, with water, to a sucrose syrup from 78.0 to 82.0º Brix and heated at 80º for total dissolution of sucrose, through agitation in the tank. That syrup is then cooled down to 55.0 to 60.0 ºC, and the pH adjusted at 4.5 using citric acid. The invertase enzyme, either GMO-free or commercially available, is added to the syrup, and the reaction is kept for the time required to achieve inversion of up to 98.0% of the sucrose into glucose and fructose under the described conditions. The syrup may be added with fibers, vitamins, minerals, amino acids, aroma identical to the natural flavor, artificial aroma, and optionally natural aroma. Depending on the sucrose source, the product color may be adjusted by using natural colorants. On top of these additives, the artificial honey product may also be added with other aromatizers, such as orange blossom, truffle, pepper, etc.

The product obtained from this process exhibits very similar characteristics than those of bee honey in terms of appearance, thickness, and flavor. This product has been used for a comparison with Manuka honey and natural bee honey. **Table 3** shows the results of the comparison, using, for example, artificial sugar Demerara, bee honey, and Manuka honey ***[***Ajibola A, Chamunorwa JP, Erlwanger KH. Nutraceutical values of natural honey and its contribution to human health and wealth. Nutrition & Metabolism 2012, 9:61***;*** Moniruzzaman M, Chowdhury MAZ, Rahman MA, Sulaiman SA, Gan SH. Determination of mineral, trace element, and pesticide levels in honey samples originating from different regions of Malaysia compared to Manuka Honey. Biomed. Res. Int. 2014, ID 359890***;*** Nogueira FS, Ferreira KS, Carneiro Junior JB, Passoni LC. Minerais em melados e em caldos de cana. Ciência e Tecnologia de Alimentos 2009, 29:727-731***;*** Silva AFS. Caracterização e determinação de minerais em amostras de açúcares brasileiros. Master's Thesis. ESALQ, USP, Piracicaba, 2017***].***

**Table 3: Composition comparison between bee honey and artificial honey Demerara.**

| **Components** | **Bee honey (%)** | **Artificial honey (%)** | **Manuka** |
|---|---|---|---|
| Water | 17.2 | 18.0 | 15 |
| Fructose | 38.2 | 39.0 | **Carbohydrates** 85% |
| Glucose | 31.3 | 40.0 | |
| sucrose | 0.7 | 2.0 | |
| others | 9.5 | - | - |
| Total minerals | 0.2 | 0.3 | - |
| Total fibers | 0 | 10.0 | - |
| Amino Acids and Proteins | 0.3 | - | 1% |
| Sodium mg/100g | 1.6 to 17 | 0.91 | 19.6 |
| Zinc mg/100g | 0.05 to 2 | 0.009 - 0.036 | 15.8 |
| Potassium mg/100g | 40 to 3500 | 1.4 to 4.9 | 122.9 |
| Magnesium mg/100g | 0.7 to 13 | 1.2 to 7.9 | 4.5 |
| Calcium mg/100g | 3 to 31 | 8.0 to 24.4 | 80.9 |
| Iron mg/100g | 0.03 to 4 | 0.3 to 5.8 | 21.7 |
| Complex-B vitamins mg/100g | 0.01 to 0.32 | 0.01 to 0.03 | Trace |

As per the studies conducted, when we use the above-described process, it is possible to obtain an artificial honey product with the following advantages when compared to bee honey:
- Sustainable process, contributing toward the preservation of bees and, as a consequence, cooperating in the pollination process that is key in farming activities;
- Source of fibers. Adding fibers is an important aspect, since it allows for a slower digestion of carbohydrates, avoiding insulin peaks and improving the gut flora. It is worth lighting that natural honey does not contain fibers;
- Free of microorganisms. Bee honey usually has the typical microorganisms found in bees, which may pose a pathogenic risk. For that reason, giving honey to very young children is not advisable, since they do not have a complete immune system to fight such risks;
- Pharmacological potential preserved due to the use of brown sugars, such as VHP or Demerara sugars, which have pharmacologically active compounds in their natural composition.

However, this invention has verified that the artificial honey product, with these and other advantages, may be obtained from slight variations in the above-described process with different types of sucrose. Thus, in order to better illustrate the different ways of obtaining it and the types of sucrose, below are illustrative examples of processes and composition of the artificial honey product of this invention, considering that the values shown in the tables are based on ***[***Ajibola A., Chamunorwa JP, Erlwanger KH. Nutraceutical values of natural honey and its contribution to human health and wealth. Nutrition & Metabolism 2012, 9:61***;*** Faria DAM. Estudo Nutricional e sensorial de açúcares cristal, refinado, demerara e mascavo orgânicos e convencionais. Master's Thesis. UFSCAR, 2012***;*** Luchini DL. Teores de nutrientes minerais e metais pesados em açúcar mascavo produzido por diferentes sistemas orgânicos e convencionais. Master's Thesis. UFSCAR, 2014***;*** Moniruzzaman M, Chowdhury MAZ, Rahman MA, Sulaiman SA, Gan SH. Determination of mineral, trace element, and pesticide levels in honey samples originating from different regions of Malaysia compared to Manuka Honey. Biomed. Res. Int. 2014, ID 359890***;*** Nogueira FS, Ferreira KS, Carneiro Junior JB, Passoni LC. Minerais em melados e em caldos de cana. Ciência e Tecnologia de Alimentos 2009, 29:727-731***;*** Silva AFS. Caracterização e determinação de minerais em amostras de açúcares brasileiros. Master's Thesis. ESALQ, USP, Piracicaba, 2017***].***

### Example 1 - Artificial honey production from coarse sugarcane inverted ENZYMATICALLY:

1. Solid sucrose is diluted with water to a sucrose syrup from 78.0 to 82.0 ºBrix and heated at 80 ºC for total dissolution of the sugar, through agitation in the tank;
2. The syrup is cooled down to 55.0 to 60.0 ºC, and the pH adjusted at 4.5 using citric acid. In that syrup, the invertase enzyme (whether or not GMO-free) is added as indicated for commercial applications, and the reaction is kept for the time required to ensure the necessary inversion percentage;
3. The syrup is filtered for removal of particulates;
4. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma;

Thus, **example 1** refers to:
✔ using **COARSE SUGAR** (organic, non-organic, or refined) as the basis for the artificial honey product of this invention;
✔ **ENZYMATICALLY** inverted at different inversion rates (sucrose/glucose+fructose ratio: up to 98% inversion);
✔ aromatized with a honey aroma (natural, artificial, or identical to the natural flavor).

The product is similar to bee honey in terms of appearance, thickness, and flavor, as shown in Table 4.

**Table 4: Example comparing the composition of bee honey and artificial honey from coarse sugar.**

| **Components** | **Bee honey** | **Coarse Sugar artificial honey** |
|---|---|---|
| Water (%) | 15.0 - 20.0 | 18.0 - 20.0 |
| Fructose (%) | 30,0 - 45,0 | 0 - 49.0 |
| Glucose (%) | 24.0 - 40.0 | 0 - 49.0 |
| Sucrose (%) | 0.1 - 4.8 | 2.0 - 100.0 |
| Others (%) | 2.0 - 8.0 | 0 |
| Total fibers (%) | 0 | 0 |
| Amino Acids and Proteins (mg/100g) | 0.2 - 0.4 | 0.1 |
| Sodium mg/100g | 1.6 - 17.0 | 0.6 - 0.9 |
| Zinc mg/100g | 0.05- 2.0 | 0.007 - 0,1 |
| Potassium mg/100g | 40.0 - 3500.0 | 0.47- 8.1 |
| Magnesium mg/100g | 0.7 - 13 | 0.26 - 3.25 |
| Calcium mg/100g | 3.0-31.0 | 10.6 - 121.4 |
| Iron mg/100g | 0.03 - 4.0 | 0.08 - 0.9 |

### Example 2 - Artificial honey production from coarse sugarcane sugar inverted CHEMICALLY:

1. Solid sucrose is diluted with water to a sucrose syrup from 78.0 to 82.0 ºBrix and heated at 80 ºC for total dissolution of the sugar, through intense agitation in the tank:
   - Phosphoric acid is added to the syrup up to pH 2.0 to 2,5, keeping it at 95 ºC until the desired inversion rate;
2. The syrup pH is corrected with soda ash to pH 4.5 to 5,0;
3. The syrup is filtered for removal of particulates;
4. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma. Thus, **example 2** refers to:
   ✔ using **COARSE SUGAR** (organic, non-organic, or refined) as the basis for the artificial honey product, as shown in example 1, however not using an enzyme;
   ✔ **CHEMICALLY** inverted at different inversion rates (sucrose/glucose+fructose ratio from 0 to 98% inversion);
   ✔ aromatized with a honey aroma (natural, artificial, or identical to the natural scent).

The product is similar to bee honey in terms of appearance, thickness, and flavor, as shown in Table 5.

**Table 5: Example comparing the composition of bee honey and artificial honey from coarse sugar.**

| **Components** | **Bee honey** | **Coarse Sugar artificial honey** |
|---|---|---|
| Water (%) | 15.0 - 20.0 | 18 - 20.0 |
| Fructose (%) | 30.0 - 45.0 | 0 - 49.0 |
| Glucose (%) | 24.0 - 40.0 | 0 - 49.0 |
| Sucrose (%) | 0.1 -4.8 | 2.0 - 100.0 |
| Others (%) | 2.0 - 8.0 | 0 |
| Total fibers (%) | 0 | 0 |
| Amino Acids and Proteins (%) | 0.2 - 0.4 | 0.1 |
| Sodium mg/100g | 1.6 - 17.0 | 0.6 - 0.9 |
| Zinc mg/100g | 0.05- 2.0 | 0.007 - 0,1 |
| Potassium mg/100g | 40.0 - 3500.0 | 0.47-8.1 |
| Magnesium mg/100g | 0.7 - 13 | 0.26 - 3.25 |
| Calcium mg/100g | 3.0 - 31.0 | 10.6 - 121.4 |
| Iron mg/100g | 0.03 - 4.0 | 0.08 - 0.9 |

It is worth stressing that, with a sucrose inversion using the traditional chemical method, the initial syrup has a maximum concentration of 60 ºBrix due to the need for filtrations to remove color and odor, and, after the inversion, the syrup pH needs to be corrected using soda ash, a fact that saturates the syrup with undesirable sulfates, and then the syrup is filtered and concentrated on evaporators, which further reduces its quality due to the additional build-up of undesirable substances such as: furfural, hydromethylfurfural, and mainly sulfooxymethylfurfural, given its capacity to react to the DNA and cause mutations ***[***Ogando FIB. Estudo da degradação térmica de sacarose e da contaminação microbiológica no processo de fabricação de açúcar. Master's Theses, ESALQ/USP, 2015***].***

### Example 3 - Artificial honey production from coarse sugarcane sugar inverted CHEMICALLY following the Positive List of Organics:

1. Solid sucrose is diluted with water to a sucrose syrup from 78.0 to 82.0 ºBrix and heated at 80 ºC for total dissolution of the sugar, through intense agitation in the tank:
2. Acid is added to the syrup up to pH 2.0 to 2,5, keeping it at 95 ºC until the desired inversion rate. Acid is used in this process must be allowed on the Positive List of Organics (executive instruction No. 18, dated May 28, 2009, Law 10831, dated December 23, 2003); example: citric acid;
3. The syrup pH is corrected with products allowed on the Positive List of Organics (executive instruction No. 18, dated May 28, 2009, Law 10831, dated December 23, 2003); to pH 4.5 to 5.0;
4. The syrup is filtered for removal of particulates;
5. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma.

Thus, **example 3** refers to:
✔ use of **ORGANIC COARSE SUGAR** based on artificial honey, as shown in example 2, but using the positive list of acids;
✔ **CHEMICALLY** inverted with products allowed on the Positive List of Organics at different inversion rates (sucrose/glucose+fructose ratio from 0 to 98% inversion), regarded as an organic syrup;
✔ aromatized with a honey aroma (natural, artificial, or identical to the natural scent).

The product is similar to bee honey in terms of appearance, thickness, and flavor, as shown in Table 6.

**Table 6: Example comparing the composition of bee honey and artificial honey from coarse sugar.**

| **Components** | **Bee honey** | **Coarse Sugar artificial honey** |
|---|---|---|
| Water (%) | 15.0 - 20.0 | 18 - 20.0 |
| Fructose (%) | 30.0 - 45.0 | 0 - 49.0 |
| Glucose (%) | 24.0 - 40.0 | 0 - 49.0 |
| Sucrose (%) | 0.1 -4.8 | 2.0 -100.0 |
| Others (%) | 2.0 - 8.0 | 0 |
| Total fibers (%) | 0 | 0 |
| Amino Acids and Proteins (%) | 0.2 - 0.4 | 0.1 |
| Sodium (mg/100g) | 1.6 - 17.0 | 0.6 - 0.9 |
| Zinc (mg/100g) | 0.05- 2.0 | 0.007 - 0,1 |
| Potassium (mg/100g) | 40.0 - 3500.0 | 0.47-8.1 |
| Magnesium (mg/100g) | 0.7 - 13 | 0.26 - 3.25 |
| Calcium (mg/100g) | 3.0 - 31.0 | 10.6 - 121.4 |
| Iron (mg/100g) | 0.03 - 4.0 | 0.08 - 0.9 |

### Example 4 - Artificial honey production from VHP or VVHP sugar:

1. Solid VVHP (Very Very High Polarization) or VHP (Very High Polarization) sucrose is diluted with water to a sucrose syrup from 78.0 to 82.0 ºBrix and heated at 80 ºC for total dissolution of the sugar, through agitation in the tank;
2. The syrup is **CHEMICALLY** inverted (organic or non-organic products) or **ENZYMATICALLY** as described in the previous examples;
3. The syrup is filtered for removal of particulates;
4. The filtered syrup is added with: (i) aroma identical to the natural flavor (example: aroma AIN Artificial Honey/Grasse), (ii) artificial honey aroma, or
   (iii) optionally, natural honey aroma. The aroma proportion indicated in this product is 0.1% to 5.0% (w/w) or until it becomes identical to the natural honey aroma.

Thus, **example 4** refers to:
✔ use of **VHP ou VVHP** sugars (organic or not) as a basis for the artificial honey product;
✔ **CHEMICALLY** or **ENZYMATICALLY** inverted at different inversion rates (sucrose/glucose+fructose ratio from 0 to 98% inversion);
✔ aromatized with a honey aroma (natural, artificial, or identical to the natural scent).

The product is similar to bee honey in terms of appearance, thickness, and flavor, as shown in Table 7.

**Table 7: Example comparing the composition of bee honey and artificial honey from inverted VHP or VVHP sugars:**

| **Components** | **Bee honey** | **VHP artificial honey** |
|---|---|---|
| Water (%) | 15.0 - 20.0 | 18 - 20.0 |
| Fructose (%) | 30.0 - 45.0 | 0 - 49.0 |
| Glucose (%) | 24.0 - 40.0 | 0 - 49.0 |
| Sucrose (%) | 0.1 - 4.8 | 2.0 -100.0 |
| Others (%) | 2.0 - 8.0 | 0 |
| Total fibers (%) | 0 | 0 |
| Amino Acids and Proteins (mg/100g) | 0.2 - 0.4 | 3.5 |
| Sodium (mg/100g) | 1.6 - 17.0 | 1.4-12.1 |
| Zinc (mg/100g) | 0.05- 2.0 | 0.07 - 0.35 |
| Potassium (mg/100g) | 40.0 - 3500.0 | 13.5-143.7 |
| Magnesium (mg/100g) | 0.7- 13 | 4.56 - 34.1 |
| Calcium (mg/100g) | 3.0 - 31.0 | 10.8- 29.5 |
| Iron (mg/100g) | 0.03 - 4.0 | 0.5-6.1 |

### Example 5 - Artificial honey production from Demerara sugar:

1. Solid Demerara sucrose is diluted with water to a sucrose syrup from 78.0 to 82.0 ºBrix and heated at 80 ºC for total dissolution of the sugar, through agitation in the tank;
2. The syrup is **CHEMICALLY** inverted (organic or non-organic products) or **ENZYMATICALLY** as described in the previous examples;
3. The syrup is filtered for removal of particulates;
4. The filtered syrup is added with: (i) aroma identical to the natural flavor (example: aroma AIN Artificial Honey/Grasse), (ii) artificial honey aroma, or
   (iii) optionally, natural honey aroma. The aroma proportion indicated in this product is 0.1% to 5.0% (w/w) or until it becomes identical to the natural honey aroma.

Thus, **EXAMPLE 5** refers to:
✔ use of **DEMERARA** sugar (organic or not) as a basis for the artificial honey product;
✔ **CHEMICALLY** or **ENZYMATICALLY** inverted at different inversion rates (sucrose/glucose+fructose ratio from 0 to 98% inversion);
✔ aromatized with a honey aroma (natural, artificial, or identical to the natural scent).

The product is similar to bee honey in terms of appearance, thickness, and flavor, as shown in Table 8.

**Table 8: Example comparing the composition of bee honey and artificial honey from inverted Demerara sugar:**

| **Components** | **Bee honey** | **Demerara artificial honey** |
|---|---|---|
| Water (%) | 15.0 - 20.0 | 18-20.0 |
| Fructose (%) | 30.0 - 45.0 | 0 - 49.0 |
| Glucose (%) | 24.0 - 40.0 | 0 - 49.0 |
| Sucrose (%) | 0.1-4.8 | 2.0-100.0 |
| Others (%) | 2.0 - 8.0 | 0 |
| Total fibers (%) | 0 | 0 |
| Amino Acids and Proteins (%) | 0.2 - 0.4 | 3.5 |
| Sodium (mg/100g) | 1.6-17.0 | 9.3 |
| Zinc (mg/100g) | 0.05- 2.0 | 0.009 - 0.036 |
| Potassium (mg/100g) | 40.0 - 3500.0 | 1.4 - 4.9 |
| Magnesium (mg/100g) | 0.7 - 13 | 1.2 - 7.9 |
| Calcium (mg/100g) | 3.0 - 31.0 | 8.02 - 24.42 |
| Iron (mg/100g) | 0.03 - 4.0 | 0.33 - 5.78 |

### Example 6 - Artificial honey production from the sugars: Coarse, VHP, VVHP, or Demerara enriched with soluble fibers:

1. Solid sucrose from any type of sugar is diluted with water to a sucrose syrup from 78.0 to 82.0 ºBrix and heated at 80 ºC for total dissolution of the sugar, through agitation in the tank;
2. The syrup is **CHEMICALLY** inverted (organic or non-organic products) or **ENZYMATICALLY** as described in the previous examples;
3. The syrup is filtered for removal of particulates;
4. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma;
5. The honey syrup is added with 0.5% to 20.0% of soluble fibers, organic or not, such as, for instance: corn fibers, polydextroses, soluble maltodextrins (example: Promitor 70R/Grasse), or cassava fibers (example: *LowPure* Tapioca *900*/*Gramkow).*

Thus, **example 6** refers to:
✔ use of the organic or non-organic sugars: **Coarse, VHP, VVHP,** or **DEMERARA** as a basis for the artificial honey product;
✔ **CHEMICALLY** or **ENZYMATICALLY** inverted at different inversion rates (sucrose/glucose+fructose ratio from 0 to 98% inversion);
✔ aromatized with a honey aroma (natural, artificial, or identical to the natural scent);
✔ Enriched with **SOLUBLE FIBERS.**

The product is similar to bee honey in terms of appearance, thickness, and flavor, but it is rich in fibers, as shown in Table 9.

**Table 9: Example comparing the composition of bee honey and artificial honey. Comparison based on enzymatically inverted DEMERARA sugar rich in soluble fibers.**

| **Components** | **Bee honey** | **Demerara artificial honey** |
|---|---|---|
| Water (%) | 15.0 - 20.0 | 18 - 20.0 |
| Fructose (%) | 30.0 - 45.0 | 0 - 49.0 |
| Glucose (%) | 24.0 - 40.0 | 0 - 49.0 |
| Sucrose (%) | 0.1-4.8 | 2.0-100.0 |
| Others (%) | 2.0 - 8.0 | 0 |
| Total fibers (%) | 0 | 0.5 - 20 |
| Amino Acids and Proteins (%) | 0.2 - 0.4 | 3.5 |
| Sodium (mg/100g) | 1.6-17.0 | 9.3 |
| Zinc (mg/100g) | 0.05- 2.0 | 0.009 - 0.036 |
| Potassium (mg/100g) | 40.0 - 3500.0 | 1.4 - 4.9 |
| Magnesium (mg/100g) | 0.7 - 13 | 1.2 - 7.9 |
| Calcium (mg/100g) | 3.0 - 31.0 | 8.02 - 24.42 |
| Iron (mg/100g) | 0.03 - 4.0 | 0.33 - 5.78 |

### Example 7 - Artificial honey production from the sugars: Coarse, VHP, VVHP, or Demerara enriched with insoluble fibers:

1. Solid sucrose from any type of sugar is diluted with water to a sucrose syrup from 78.0 to 82.0 ºBrix and heated at 80 ºC for total dissolution of the sugar, through agitation in the tank;
2. The syrup is **CHEMICALLY** inverted (organic or non-organic products) or **ENZYMATICALLY** as described in the previous examples;
3. The syrup is filtered for removal of particulates;
4. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma;
5. The honey syrup is added with 0.5% to 20.0% organic or non-organic insoluble fibers, such as corn or cassava fibers (example: Fibervita - MF Carrier 125) or plant fibers (Example: inulin/Grasse).

Thus, **example 7** refers to:
✔ use of the organic or non-organic sugars: **Coarse, VHP, VVHP,** or **DEMERARA** as a basis for the artificial honey product;
✔ **CHEMICALLY** or **ENZYMATICALLY** inverted at different inversion rates (sucrose/glucose+fructose ratio from 0 to 98% inversion);
✔ aromatized with a honey aroma (natural, artificial, or identical to the natural scent);
✔ Enriched with **INSOLUBLE FIBERS.**

The product is similar to bee honey in terms of appearance, thickness, and flavor, but it is regarded as a food rich in insoluble fibers, as shown in Table 10.

**Table 10: Example comparing the composition of bee honey and artificial honey. Comparison based on enzymatically inverted DEMERARA sugar rich in insoluble fibers.**

| **Components** | **Bee honey** | **LIKEaB Demerara honey** |
|---|---|---|
| Water (%) | 15.0 - 20.0 | 18 - 20.0 |
| Fructose (%) | 30.0 - 45.0 | 0 - 49.0 |
| Glucose (%) | 24.0 - 40.0 | 0 - 49.0 |
| Sucrose (%) | 0.1-4.8 | 2.0-100.0 |
| Others (%) | 2.0 - 8.0 | 0 |
| Total insoluble fibers (%) | 0 | 0.5 - 20 |
| Amino Acids and Proteins (mg/100g) | 0.2 - 0.4 | 3.5 |
| Sodium (mg/100g) | 1.6-17.0 | 9.3 |
| Zinc (mg/100g) | 0.05- 2.0 | 0.009 - 0.036 |
| Potassium (mg/100g) | 40.0 - 3500.0 | 1.4 - 4.9 |
| Magnesium (mg/100g) | 0.7 - 13 | 1.2 - 7.9 |
| Calcium (mg/100g) | 3.0 - 31.0 | 8.02 - 24.42 |
| Iron (mg/100g) | 0.03 - 4.0 | 0.33 - 5.78 |

### Example 8 - Artificial honey production from the sugars: Coarse, VHP, VVHP, or Demerara enriched with soluble and insoluble fibers:

1. Solid sucrose from any type of sugar is diluted with water to a sucrose syrup from 78.0 to 82.0 ºBrix and heated at 80 ºC for total dissolution of the sugar, through intense agitation in the tank;
2. The syrup is **CHEMICALLY** inverted (organic or non-organic products) or **ENZYMATICALLY** as described in the previous examples;
3. The syrup is filtered for removal of particulates;
4. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma;
5. The syrup is added with 1.0% to 20.0% soluble or insoluble fibers in a proportion of 1.0 to 99.0% of mixture of both types of fibers.

Thus, **example 8** refers to:
✔ use of the organic or non-organic sugars: **Coarse, VHP, VVHP,** or **DEMERARA** as a basis for the artificial honey product;
✔ **CHEMICALLY** or **ENZYMATICALLY** inverted at different inversion rates (sucrose/glucose+fructose ratio from 0 to 98% inversion);
✔ aromatized with a honey aroma (natural, artificial, or identical to the natural scent);
✔ Enriched with **SOLUBLE AND INSOLUBLE FIBERS.**

The product is similar to bee honey in terms of appearance, thickness, and flavor, but it is regarded as a food rich in soluble and insoluble fibers, as shown in Table 11.

**Table 11: Example comparing the composition of bee honey and artificial honey. Comparison based on enzymatically inverted DEMERARA sugar rich in soluble and insoluble fibers:**

| **Components** | **Bee honey** | **Demerara artificial honey** |
|---|---|---|
| Water % | 15.0 - 20.0 | 18 - 20.0 |
| Fructose (%) | 30.0 - 45.0 | 0 - 49.0 |
| Glucose (%) | 24.0 - 40.0 | 0 - 49.0 |
| Sucrose (%) | 0.1-4.8 | 2.0-100.0 |
| Others (%) | 2.0 - 8.0 | 0 |
| Total soluble and insoluble fibers (%) | 0 | 0.5 - 20 |
| Amino Acids and Proteins (mg/100g) | 0.2 - 0.4 | 3.5 |
| Sodium (mg/100g) | 1.6-17.0 | 9.3 |
| Zinc (mg/100g) | 0.05- 2.0 | 0.009 - 0.036 |
| Potassium (mg/100g) | 40.0 - 3500.0 | 1.4 - 4.9 |
| Magnesium (mg/100g) | 0.7 - 13 | 1.2 - 7.9 |
| Calcium (mg/100g) | 3.0 - 31.0 | 8.02 - 24.42 |
| Iron (mg/100g) | 0.03 - 4.0 | 0.33 - 5.78 |

### Example 9 - Artificial honey production from the sugars: Coarse, VHP, VVHP, or Demerara enriched with fibers and added with minerals:

1. Solid sucrose from any type of sugar is diluted with water to a sucrose syrup from 78.0 to 82.0 ºBrix and heated at 80 ºC for total dissolution of the sugar, through agitation in the tank;
2. The syrup is **CHEMICALLY** inverted (organic or non-organic products) or **ENZYMATICALLY** as described in the previous examples;
3. The syrup is filtered for removal of particulates;
4. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma;
5. The syrup is added with 1.0 % to 20.0% with soluble or insoluble fibers or a soluble/insoluble fiber combination;
6. The syrup may receive additives taking into account the range for a daily intake of Zinc, Calcium, Phosphorus, Iron, and Magnesium as indicated for the various age groups or group of necessities according to an ANVISA regulation approved under Decree No. 3029, dated April 16, 1999, combined with article 111, item 1, letter "e" of the Bylaws approved under Ordinance No. 593, dated August 25, 2000, published in the December 22, 2000 DOU (Federal Official Gazette), at a meeting held on December 6, 2004. Other national or foreign regulations may also be used as a reference. Also, other minerals, such as copper, selenium, manganese, and phosphorus, may be added within the limits established in the various regulations.

Thus, **example 9** refers to:
✔ use of the organic or non-organic sugars: **Coarse, VHP, VVHP,** or **DEMERARA** as a basis for the artificial honey product;
✔ **CHEMICALLY** or **ENZYMATICALLY** inverted at different inversion rates (sucrose/glucose+fructose ratio from 0 to 98% inversion);
✔ aromatized with a honey aroma (natural, artificial, or identical to the natural scent);
✔ Enriched with **SOLUBLE OR INSOLUBLE FIBERS OR BOTH;**
✔ Enriched with **MINERALS.**

The product is similar to bee honey in terms of appearance, thickness, and flavor, but it is regarded as a food rich in fibers (soluble or insoluble or both) and minerals, as shown in Table 12:

**Table 12: Example comparing the composition of bee honey and artificial honey. Comparison based on enzymatically inverted DEMERARA sugar rich in soluble/insoluble fibers and minerals. Example with 100% of the Anvisa-recommended dose:**

| **Components** | **Bee honey** | **Demerara artificial honey** |
|---|---|---|
| Water (%) | 15.0 - 20.0 | 18-20.0 |
| Fructose (%) | 30.0 - 45.0 | 0 - 49.0 |
| Glucose (%) | 24.0 - 40.0 | 0 - 49.0 |
| Sucrose (%) | 0.1-4.8 | 2.0-100.0 |
| Others (%) | 2.0 - 8.0 | 0 |
| Total fibers (%) | - | 0.5 - 20.0 |
| Amino Acids and Proteins | 0.3 | 3.5 |
| Sodium (mg/100g) | 1.6 to 17 | 9.3 |
| Zinc (mg/100g/day) | 0.05 to 2 | Up to 7.0 |
| Potassium (mg/100g) | 40 - 3500 | 1.4 - 4.9 |
| Magnesium (mg/100g/day) | 0.7- 13 | Up to 260.0 |
| Calcium (mg/100g/day) | 3-31.0 | Up to 1000.0 |
| Iron (mg/100g/day) | 0.03 - 4.0 | Up to 14.0 |
| Phosphorus (mg/100g/day) | 2.0-15.0 | Up to 700.0 |

### Example 10 - Vegan artificial honey production from the sugars: Coarse, VHP, VVHP, or Demerara enriched with fibers, minerals, and vitamins:

1. Solid sucrose from any type of sugar is diluted with water to a sucrose syrup from 78.0 to 82.0 ºBrix and heated at 80 ºC for total dissolution of the sugar, through agitation in the tank;
2. The syrup is inverted **CHEMICALLY** inverted (organic or non-organic products) or **ENZYMATICALLY** as described in the previous examples;
3. The syrup is filtered for removal of particulates;
4. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma;
5. The syrup is added with 1.0 % to 20.0% with soluble or insoluble fibers or a soluble/insoluble fiber combination;
6. The syrup may be added taking into account the range for a daily intake of Zinc, Calcium, Phosphorus, Iron, and Magnesium as indicated for the various age groups or group of necessities according to an ANVISA regulation approved under Decree No. 3029, dated April 16, 1999, combined with article 111, item 1, letter "e" of the Bylaws approved under Ordinance No. 593, dated August 25, 2000, published in the December 22, 2000 DOU (Federal Official Gazette), at a meeting held on December 6, 2004. Other national or foreign regulations may also be used as a reference. Also, other minerals, such as copper, selenium, manganese, and phosphorus, may be added within the limits established in the various regulations;
7. The syrup may be added taking into account the range for a daily intake of vitamins of the A, B, C, D, and E complexes, folic acid, riboflavin, thiamine, and niacin, as indicated for the various age groups or group of necessities according to an ANVISA regulation approved under Decree No. 3029, dated April 16, 1999, combined with article 111, item 1, letter "e" of the Bylaws approved under Ordinance No. 593, dated August 25, 2000, published in the December 22, 2000 DOU (Federal Official Gazette), at a meeting held on December 6, 2004. Other national or foreign regulations may also be used as a reference. Other vitamins, such as vitamin B5 - pantothenic acid, and vitamin K, may be added according to the various regulations.

Thus, **example 10** refers to:
✔ use of the organic or non-organic sugars: **Coarse, VHP, VVHP,** or **DEMERARA** as a basis for the artificial honey product;
✔ **CHEMICALLY** or **ENZYMATICALLY** inverted at different inversion rates (sucrose/glucose+fructose ratio from 0 to 98% inversion);
✔ aromatized with a honey aroma (natural, artificial, or identical to the natural scent);
✔ Enriched with **SOLUBLE OR INSOLUBLE FIBERS OR BOTH;**
✔ Enriched with **MINERALS;**
✔ Enriched with **VITAMINS.**

The product is similar to bee honey in terms of appearance, thickness, and flavor, but it is regarded as a food rich in fibers (soluble or insoluble or both), minerals, and vitamins, as shown in Table 13:

**Table 13: Example comparing the composition of bee honey and artificial honey. Comparison based on enzymatically inverted DEMERARA sugar rich in fibers, minerals, and vitamins. Example with 100% of the Anvisa-recommended dose:**

| **Components** | **Bee honey** | **Demerara artificial honey** |
|---|---|---|
| Water (%) | 15.0 - 20.0 | 18 - 20.0 |
| Fructose (%) | 30.0 - 45.0 | 0 - 49.0 |
| Glucose (%) | 24.0 - 40.0 | 0 - 49.0 |
| Sucrose (%) | 0.1-4.8 | 2.0-100.0 |
| Others (%) | 2.0 - 8.0 | 0 |
| Total fibers (%) | - | 0.5 - 20.0 |
| Amino Acids and Proteins | 0.3 | 3.5 |
| Sodium (mg/100g) | 1.6 to 17 | 9.3 |
| Zinc (mg/100g/day) | 0.05 to 2 | Up to 5.6 |
| Potassium (mg/100g) | 40 - 3500 | 1.4 - 4.9 |
| Magnesium (mg/100g/day) | 0.7-13 | Up to 208.0 |
| Calcium (mg/100g/day) | 3-31.0 | Up to 800.0 |
| Iron (mg/100g/day) | 0.03 - 4.0 | Up to 11.2 |
| Phosphorus (mg/100g/day) | 2.0-15.0 | Up to 560.0 |
| Vitamin A (mg/100g/day) | 0 | Up to 0.6 |
| B1 - Thiamine (mg/100g/day) | 0.01 | Up to 1.2 |
| B2 - Riboflavin (mg/100g/day) | 0.01 - 0.02 | Up to 1.3 |
| B3 - Niacin (mg/100g/day) | 0.1 - 0.2 | Up to 16.0 |
| B6 - Pyridoxine (mg/100g/day) | 0.01 - 0.32 | Up to 1.3 |
| B9 - Folic acid (mg/100g/day) | 0.002 - 0.01 | Up to 0.4 |
| Vitamins C (mg/100g/day) | 2.2 - 2.5 | Up to 45.0 |
| Vitamins D (mg/100g/day) | 0 | Up to 0.005 |
| Vitamins E (mg/100g/day) | 0 | Up to 10.0 |

### Example 11 - Artificial honey production from the sugars: Coarse, VHP, VVHP, or Demerara enriched with fibers, minerals, and vitamins and amino acids:

1. Solid sucrose from any type of sugar is diluted with water to a sucrose syrup from 78.0 to 82.0 ºBrix and heated at 80 ºC for total dissolution of the sugar, through intense agitation in the tank;
2. The syrup is **CHEMICALLY** inverted (organic or non-organic products) or **ENZYMATICALLY** as described in the previous examples;
3. The syrup is filtered for removal of particulates;
4. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma;
5. The syrup is added with 1.0 % to 20.0% with soluble or insoluble fibers or a soluble/insoluble fiber combination;
6. The syrup may be added taking into account the range for a daily intake of Zinc, Calcium, Phosphorus, Iron, and Magnesium as indicated for the various age groups or group of necessities according to an ANVISA regulation approved under Decree No. 3029, dated April 16, 1999, combined with article 111, item 1, letter "e" of the Bylaws approved under Ordinance No. 593, dated August 25, 2000, published in the December 22, 2000 DOU (Federal Official Gazette), at a meeting held on December 6, 2004. Other national or foreign regulations may also be used as a reference. Also, other minerals, such as copper, selenium, manganese, and phosphorus, may be added within the limits established in the various regulations;
7. The syrup may be added taking into account the range for a daily intake of vitamins of the A, B, C, D, and E complexes, folic acid, riboflavin, thiamine, and niacin, as indicated for the various age groups or group of necessities according to an ANVISA regulation approved under Decree No. 3029, dated April 16, 1999, combined with article 111, item 1, letter "e" of the Bylaws approved under Ordinance No. 593, dated August 25, 2000, published in the December 22, 2000 DOU (Federal Official Gazette), at a meeting held on December 6, 2004. Other national or foreign regulations may also be used as a reference. Other vitamins, such as vitamin B5 - pantothenic acid, and vitamin K, may be added according to the various regulations;
8. According to the characteristics of the various types of natural honey (flowers, region, seasonality, etc.), the syrup may also be added with various types of amino acids, such as: glutamic acid, aspartic acid, glutamine, histidine, glycine, threonine, alanine, arginine, proline, tyrosine, valine, methionine, cysteine, leucine, phenylalanine, isoleucine, tryptophan, ornithine, and lysine, and others. However, as said before, either the presence or the absence of those amino acids in natural honey depends on the place of occurrence, type of flower, type of pollen, etc. However, in the artificial honey product of this invention, we can optionally add a mix of amino acids that will mimic or is superior to the composition of conventional honey. Thus, the artificial honey product can have superior standardization, always making sure that, in the end product, the various amino acids can be present.

Thus, the claim in **example 11** refers to:
✔ use of the organic or non-organic sugars: **Coarse, VHP, VVHP,** or **DEMERARA** as a basis for the artificial honey product;
✔ **CHEMICALLY** or **ENZYMATICALLY** inverted at different inversion rates (sucrose/glucose+fructose ratio from 0 to 98% inversion);
✔ aromatized with a honey aroma (natural, artificial, or identical to the natural scent);
✔ Enriched with **SOLUBLE OR INSOLUBLE FIBERS OR BOTH;**
✔ Enriched with **MINERALS;**
✔ Enriched with **VITAMINS;**
✔ Enriched with **AMINO ACIDS.**

The product is similar to bee honey in terms of appearance, thickness, and flavor, but it is regarded as a food rich in fibers, minerals, vitamins, and amino acids, as shown in Table 14:

**Table 14: Example comparing the composition of bee honey and artificial honey. Comparison based on enzymatically inverted DEMERARA sugar rich in fibers, minerals, vitamins, and amino acids. Example with 1.0 to 2.0% amino acids, according to Alvares et al, 2010 [Alvarez-Suarez JM et al. Contribution of honey in nutrition and human health: a review. Mediterr J Nutr Metab 2010, 3:15-23.**

| **Components** | **Bee honey** | **Demerara artificial honey** |
|---|---|---|
| Water (%) | 15.0 - 20.0 | 18 - 20.0 |
| Fructose (%) | 30.0 - 45.0 | 0 - 49.0 |
| Glucose (%) | 24.0 - 40.0 | 0 - 49.0 |
| Sucrose (%) | 0.1 - 4.8 | 2.0 - 100.0 |
| Others (%) | 2.0 - 8.0 | 0 |
| Total fibers (%) | - | 0.5 - 20.0 |
| Amino Acids and Proteins (mg/100g) | 0.2-0.4 | 1.0 - 2.0 |
| Sodium (mg/100g) | 1.6 to 17 | 9.5 |
| Zinc (mg/100g/day) | 0.05 to 2 | Up to 5.6 |
| Potassium (mg/100g) | 40 - 3500 | 1.4 - 4.9 |
| Magnesium (mg/100g/day) | 0.7- 13 | Up to 208.0 |
| Calcium (mg/100g/day) | 3-31.0 | Up to 800.0 |
| Iron (mg/100g/day) | 0.03 - 4.0 | Up to 11.2 |
| Phosphorus (mg/100g/day) | 2.0-15.0 | Up to 560.0 |
| Vitamin A (mg/100g/day) | 0 | Up to 0.6 |
| B1 - Thiamine (mg/100g/day) | 0.01 | Up to 1.2 |
| B2 - Riboflavin (mg/100g/day) | 0.01 - 0.02 | Up to 1.3 |
| B3 - Niacin (mg/100g/day) | 0.1 - 0.2 | Up to 16.0 |
| B6 - Pyridoxine (mg/100g/day) | 0.01 - 0.32 | Up to 1.3 |
| B9 - Folic acid (mg/100g/day) | 0.002 - 0.01 | Up to 0.4 |
| Vitamins C (mg/100g/day) | 2.2 - 2.5 | Up to 45.0 |
| Vitamins D (mg/100g/day) | 0 | Up to 0.005 |
| Vitamins E (mg/100g/day) | 0 | Up to 10.0 |

### Example 12 - Artificial honey production from the sugars: Coarse, VHP, VVHP, or Demerara enriched with fibers, minerals, vitamins, amino acids, and encapsulated substances:

1. Solid sucrose from any type of sugar is diluted with water to a sucrose syrup from 78.0 to 82.0 ºBrix and heated at 80 ºC for total dissolution of the sugar, through intense agitation in the tank;
2. The syrup is inverted **CHEMICALLY** inverted (organic or non-organic products) or **ENZYMATICALLY** as described in the previous examples;
3. The syrup is filtered for removal of particulates;
4. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma;
5. The syrup is added with 1.0 % to 20.0% with soluble or insoluble fibers or a soluble/insoluble fiber combination;
6. The syrup may be added taking into account the range for a daily intake of Zinc, Calcium, Phosphorus, Iron, and Magnesium as indicated for the various age groups or group of necessities according to an ANVISA regulation approved under Decree No. 3029, dated April 16, 1999, combined with article 111, item 1, letter "e" of the Bylaws approved under Ordinance No. 593, dated August 25, 2000, published in the December 22, 2000 DOU (Federal Official Gazette), at a meeting held on December 6, 2004. Other national or foreign regulations may also be used as a reference. Also, other minerals, such as copper, selenium, manganese, and phosphorus, may be added within the limits established in the various regulations;
7. The syrup may be added taking into account the range for a daily intake of vitamins of the A, B, C, D, and E complexes, folic acid, riboflavin, thiamine, and niacin, as indicated for the various age groups or group of necessities according to an ANVISA regulation approved under Decree No. 3029, dated April 16, 1999, combined with article 111, item 1, letter "e" of the Bylaws approved under Ordinance No. 593, dated August 25, 2000, published in the December 22, 2000 DOU (Federal Official Gazette), at a meeting held on December 6, 2004. Other national or foreign regulations may also be used as a reference. Other vitamins, such as vitamin B5 - pantothenic acid, and vitamin K, may be added according to the various regulations;
8. According to the characteristics of the various types of natural honey (flowers, region, seasonality, etc.), the syrup may also be added with various types of amino acids, such as: glutamic acid, aspartic acid, glutamine, histidine, glycine, threonine, alanine, arginine, proline, tyrosine, valine, methionine, cysteine, leucine, phenylalanine, isoleucine, tryptophan, ornithine, and lysine, and others. However, as said before, either the presence or the absence of those amino acids in natural honey depends on the place of occurrence, type of flower, type of pollen, etc. However, in the artificial honey product of this invention, we can optionally add a mix of amino acids that will mimic or is superior to the composition of conventional honey. Thus, the artificial honey product can have superior standardization, always making sure that, in the product, the various amino acids can be present;
9. The artificial honey product can be added with encapsulated, micro-encapsulated, or nano-encapsulated substances, for instance, sugarcane molasse, sugarcane honey, pharmacological and nutraceutical compounds, natural plant extracts, but not limited to these. The encapsulation process preserves the pharmacological properties of products and masks their potential interferences in the organoleptic properties of artificial honey; however, it maintains their functional properties intact. The addition proportion will depend on the type of product to be added and the concentration of the substances of interest, between 1.0 to 10.0% of the end honey product, though it can be higher. Some additives like vitamins, amino acids, and minerals may also be encapsulated.

Thus, the claim in **example 12** refers to:
✔ use of the organic or non-organic sugars: **Coarse, VHP, VVHP,** or **DEMERARA** as a basis for the artificial honey product;
✔ **CHEMICALLY** or **ENZYMATICALLY** inverted at different inversion rates (sucrose/glucose+fructose ratio from 0 to 98% inversion);
✔ aromatized with a honey aroma (natural, artificial, or identical to the natural scent);
✔ Enriched with **SOLUBLE OR INSOLUBLE FIBERS OR BOTH;**
✔ Enriched with **MINERALS;**
✔ Enriched with **VITAMINS;**
✔ Enriched with **AMINO ACIDS.**
✔ Enriched with **ENCAPSULATED FIBERS.**

The product is similar to bee honey in terms of appearance, thickness, and flavor, but it is regarded as a food rich in fibers, minerals, vitamins, amino acids, and encapsulated substances of interest, as shown in Table 15:

**Table 15: Example comparing the composition of bee honey and artificial honey. Comparison based on enzymatically inverted DEMERARA sugar rich in fibers, minerals, vitamins, amino acids, and encapsulated substances:**

| **Components** | **Bee honey** | **Demerara artificial honey** |
|---|---|---|
| Water (%) | 15.0 - 20.0 | 18 - 20.0 |
| Fructose (%) | 30.0 - 45.0 | 0 - 49.0 |
| Glucose (%) | 24.0 - 40.0 | 0 - 49.0 |
| Sucrose (%) | 0.1-4.8 | 2.0 - 100.0 |
| Others (%) | 2.0 - 8.0 | 0 |
| Total fibers (%) | - | 0.5 - 20.0 |
| Amino Acids and Proteins (mg/100g) | 0.2-0.4 | 1.0 - 2.0 |
| Encapsulated substances (%) | - | 1.0 - 10.0 |
| Sodium (mg/100g) | 1.6 to 17 | 9.5 |
| Zinc (mg/100g/day) | 0.05 to 2 | Up to 5.6 |
| Potassium (mg/100g) | 40 - 3500 | 1.4 - 4.9 |
| Magnesium (mg/100g/day) | 0.7- 13 | Up to 208.0 |
| Calcium (mg/100g/day) | 3-31.0 | Up to 800.0 |
| Iron (mg/100g/day) | 0.03 - 4.0 | Up to 11.2 |
| Phosphorus (mg/100g/day) | 2.0-15.0 | Up to 560.0 |
| Vitamin A (mg/100g/day) | 0 | Up to 0.6 |
| B1 - Thiamine (mg/100g/day) | 0.01 | Up to 1.2 |
| B2 - Riboflavin (mg/100g/day) | 0.01 - 0.02 | Up to 1.3 |
| B3 - Niacin (mg/100g/day) | 0.1 - 0.2 | Up to 16.0 |
| B6 - Pyridoxine (mg/100g/day) | 0.01 - 0.32 | Up to 1.3 |
| B9 - Folic acid (mg/100g/day) | 0.002 - 0.01 | Up to 0.4 |
| Vitamins C (mg/100g/day) | 2.2 - 2.5 | Up to 45.0 |
| Vitamins D (mg/100g/day) | 0 | Up to 0.005 |
| Vitamins E (mg/100g/day) | 0 | Up to 10.0 |

### Example 13 - Artificial honey production from beetroot sugar, coconut sugar, and other sugar products:

1. Sugar from beetroot, coconut, and other products is diluted with water to a sucrose syrup from 78.0 to 82.0 ºBrix and heated at 80 ºC for total dissolution of the sugar, through intense agitation in the tank;
2. The syrup is inverted **CHEMICALLY** inverted (organic or non-organic products) or **ENZYMATICALLY** as described in the previous examples;
3. The syrup is filtered for removal of particulates;
4. The filtered syrup is added with: (i) aroma identical to the natural flavor (example: aroma AIN Artificial Honey/Grasse), (ii) artificial honey aroma, or (iii) optionally, natural honey aroma. The aroma proportion indicated in this product is 0.1% to 5.0% (w/w) or until it becomes identical to the natural honey aroma;
5. The syrup may be added with fibers, minerals, vitamins, amino acids, and encapsulated substances, as shown in example 6 through 12.

The product is similar to bee honey in terms of appearance, thickness, and flavor, but it is regarded as a food rich in fibers (soluble or insoluble or both), minerals, amino acids, and encapsulated substances.

Thus, **example 12** refers to:
✔ use of beetroot sugar, organic or not, as a basis for the artificial honey product;
✔ **CHEMICALLY** or **ENZYMATICALLY** inverted at different inversion rates (sucrose/glucose+fructose ratio from 0 to 98% inversion);
✔ aromatized with a honey aroma (natural, artificial, or identical to the natural scent);
✔ It may be enriched with **FIBERS; MINERALS; VITAMINS, AMINO ACIDS, AND ENCAPSULATED SUBSTANCES.**

### Example 14 - Artificial honey production from the sugars in examples 1 through 13, with the syrup possibly added with calcium alginate and encapsulating substances aiming at encapsulation and production of artificial honey pearls.

The artificial honey product can be encapsulated using several products, such as calcium alginate, maltodextrin, and modified starches producing capsules or pearls of several diameters, not limited to only these encapsulation agents.

For instance, calcium alginate, which is the main gel used for encapsulation, because of its gelling properties, low cost, handiness, and inexistent toxicity, will be described below:
1. The honey syrup may be added with 5.0% (w/w) acid alginate heated at 70 ºC. The concentration of alginate may vary between 1.0 and 20.0% in the product;
2. The honey syrup is dripped into a water-based solution containing 3.5% CaCl₂ through agitation at 100 rpm, using mechanical dripping devices that can drip several volumes, consequentially producing sugar pearls of several diameters;
3. The pearls can be sunk in the solution for different times, as one might want a softer or harder pearl, depending on the application.

Thus, the claim in **EXAMPLE 14** refers to:
✔ use of the encapsulation technique for production of nano-, micro-, and macro-balls and pearls of honey syrup;
✔ use of encapsulating products, such as calcium alginate, maltodextrin, and modified starches, but not limited to these, for production of encapsulated artificial honey.

The honey pearls have a bee honey favor in a format never seen before on the market, thus bringing an innovation in the form and marketing of the product.

### Example 15 - Artificial honey production from the sugars in examples 1 through 13, with the honey syrup possibly added with thickeners and gelling agents with aims to obtain artificial honey gels and pastes.

The artificial honey product may be thickened using several hydrocolloids, such as Xantan gum, Guar gum, pectins, gelatins, gellan gum, carrageenans, cellulose compounds, and others, like modified starches, gelling agents, and emulsifiers, producing artificial honey gels and pastes with several textures, not limited to only said agents.

As an example, sodium carboxymethyl cellulose, which was used in the honey syrup, with a texturized honey gel being obtained, according to the example:
1. The honey syrup may be added with 1.0% (w/w) sodium carboxymethyl cellulose, such as the product Walocel CRT 40000PA. The concentration of sodium carboxymethyl cellulose may vary between 0.1 and 20.0% in the product;
2. Sodium carboxymethyl cellulose can be added directly to the honey syrup and mechanically homogenized, producing a honey product with gelatinous texture.

Thus, the claim in **EXAMPLE 15** refers to:
✔ use of thickeners, gelling agents, emulsifiers, hydrocolloids, modified starches, and cellulose compounds to obtain texturized artificial honey gels and pastes;

The honey gels and pastes have a bee honey favor in a form of supply never seen before on the market, thus bringing an innovation in the form and marketing of the product.

The above examples have been described to illustrate the various methods of production of the artificial honey product and its final composition from both types of inversion, several types of raw material, and additives, and must not be faced as limiting this invention, it being known that slight variations from the above will still be part of the scope of this invention.

In this regard, the scope of this invention also covers the various uses and applications of an artificial honey composition having such similar taste to that of natural honey with improved properties, as described in this invention. The main applications, however, without limitation, are:
a. Pharmaceutical Industry: used as a healing agent for the skin and oral formulations such as syrups and lozenges;
b. Cosmetic Industry: its emollient and healing properties can be explored by hydrants, face lotions, soaps, shampoos, and conditioners; and
c. Food Industry: its unique flavor and aroma are widely used in the food industry, such as in candies, cookies, sweets, sauces, yogurts, etc.;
d. Use as a general table product.

## Claims

1. ARTIFICIAL HONEY COMPOSITION refers to inverted sucrose, **characterized in that** it is derived from a plant source, such as sugarcane or beetroot, with conventional or organic certification, with a high degree of sucrose conversion, as high as 98% inversion, either chemically or enzymatically, exhibiting glucose and fructose concentrations very close to those of natural bee honey, without using any material or activity of animal origin, further exhibiting pharmacological, nutraceutical, and cosmetic properties present in some types of sucrose, having at least one water concentration between 10.0 to 25%; Fructose at 25.0 to 49.0%; Glucose at 25.0 to 49.0%; sucrose at 2 to 40.0%; total minerals 0.1 to 2.0%; total fibers up to 20%; total vitamins up to 0.1%, total amino acids up to 2.0%, and encapsulated products, in every 100 g of the end product.

2. COMPOSITION, as per claim 1, sucrose is **characterized in that** it is preferably a sugarcane sugar, chosen from brown sugar, VHP sugar, VVHP sugar, Demerara sugar, coarse sugar, beetroot sugar, coconut sugar, and others.

3. COMPOSITION, as per claim 1, is **characterized in that** the sugar inversion, when chemically performed, optionally uses citric acid, phosphoric acid, or even acids allowed on the Positive List, the latter case enabling the production of an organic artificial honey product.

4. COMPOSITION, as per claim 1, is **characterized in that** it contains added fructose, minerals, vitamins, fibers, natural colorants, natural aromas and extracts, and encapsulated substances for the purpose of making it as similar as possible to natural bee honey and adding improved nutraceutical, pharmacological, and organoleptic functions and properties.

5. COMPOSITION, as per claims 1 to 3, is **characterized in that** it has specific final concentrations of 17 to 21% of water; 35 to 39% of fructose; 35 to 41% of glucose; 1.0 to 2.0% of sucrose; with the optional addition of 0.2 to 2.0% of total minerals; and 0 to 5.0% of total fibers, total vitamins at 0 to 0.1%, total amino acids at 0 to 1.0%, and encapsulated products at 0 to 10.0 % in every 100 g of the end product.

6. COMPOSITION, as per claims 1 and 4, is **characterized in that** its minerals may contain the following elements in every 100 g of artificial honey: up to 17 mg of sodium; up to 7.0 mg of zinc; up to 3500 mg of potassium; up to 260 mg of magnesium; up to 1000 mg of calcium, and up to 700 mg of iron.

7. COMPOSITION, as per claim 1, **is characterized in that** the addition of the complex-B vitamin is preferably between 0.01 and 0.32 mg in every 100 g of the artificial honey product, so that the composition for final daily intake (mg/100g/day) is inside the following parameters: up to 1.2 mg of Vitamin B1; up to 1.3 mg of vitamin B2; up to 16 mg of vitamin B3; up to 1.3 mg of vitamin B6; up to 0.4 mg of vitamin B9. Moreover, the addition of other vitamins may be: up to 45 mg of vitamin C; up to 0.005 mg of vitamin D; up to 10 mg of vitamin E.

8. COMPOSITION, as per claim 1, **is characterized in that** the added fibers can be soluble and insoluble fibers, inserted in the composition either individually or jointly, producing a final food with a low glycemic index and healthier; it is produced from inverted Demerara sugar (> 98%), largely converted into glucose and fructose, with a sweetness index very close to bee honey; with added fructose or functional fibers, it exhibits a low glycemic index (<55);

9. COMPOSITION, as per claim 1, **is characterized in that** its pharmacological properties may encompass antioxidant, anti-inflammatory, anti-tumor, and antibacterial activity, depending on the sucrose used in the process.

10. COMPOSITION, as per claims 1 and 3, **characterized in that** its pharmacological activities can be performed by adding the following substances derived from the sugars chosen:
a. flavonoids, such as luteolin, apigenin, tricine, quercetin, kaempferol;
b. Phenolic, such as caffeic acid, apigenin, luteolin, tricine, chlorogenic acid, coumaric acid, ferulic acid.

11. ARTIFICIAL HONEY PRODUCTION PROCESS, to obtain the composition in the above claims, **characterized in that** it chemically or enzymatically inverts at different inversion rates (sucrose/glucose+fructose ratio from 0 to 98% inversion); contains aromatizers with a honey aroma (artificial, identical to the natural scent, and optionally a natural aroma); furthermore, it encompasses a honey syrup added with 0.5% to 20.0% soluble fibers, whether or not organic, such as corn fibers, polydextroses, soluble maltodextrins (e.g. Promitor 70R/Grasse), or cassava fibers (such LowPure Tapioca 900/Gramkow*);* a honey syrup is added with 0.5% to 20.0% insoluble fibers, whether or not organic, such as corn or cassava fibers (Fibervita - MF Carrier 125) or plant fibers (inulin/Grasse); the fact that it is added at 1.0% to 20,0% with soluble or insoluble fibers in a proportion of 1.0 to 99.0% of mixture of both types of fibers; the syrup may receive additives taking into account the range for a daily intake of Zinc, Calcium, Phosphorus, Iron, and Magnesium.

12. ARTIFICIAL HONEY PRODUCTION PROCESS, as per claim 12, **characterized in that** it can be added with encapsulated, micro-encapsulated, or nano-encapsulated substances, such as sugarcane molasse, sugarcane honey, pharmacological and nutraceutical compounds, natural plant extracts, but not limited to these; the encapsulation process preserves the pharmacological properties of products and masks their potential interferences in the organoleptic properties of artificial honey, however, it maintains their functional properties intact; the addition proportion will depend on the type of product to be added and the concentration of the substances of interest, between 1.0 to 10.0% of the end honey product, though it can be higher; some additives like vitamins, amino acids, and minerals may also be encapsulated.

13. ARTIFICIAL HONEY PRODUCTION PROCESS, as per each of the above claims, **characterized in that** it is similar to natural honey from bees, producing an artificial honey composition, whether naturally or not, however without using bees or any other animal, preserving the compounds present in the sucrose used as a raw material, and may exhibit improved functions when compared to bee honey, as well as cover the following types of production:
a. artificial honey production from coarse sugarcane sugar inverted ENZYMATICALLY;
b. artificial honey production from coarse sugarcane sugar inverted CHEMICALLY;
c. artificial honey production from organic coarse sugarcane sugar inverted CHEMICALLY following the Positive List of Organics;
d. artificial honey production from VHP or VVHP sugar;
e. artificial honey production from Demerara sugar;
f. artificial honey production from the sugars: Coarse, VHP, VVHP, or Demerara enriched with soluble fibers;
g. artificial honey production from the sugars: Coarse, VHP, VVHP, or Demerara enriched with insoluble fibers;
h. artificial honey production from the sugars: Coarse, VHP, VVHP, or Demerara enriched with soluble and insoluble fibers;
i. artificial honey production from the sugars: Coarse, VHP, VVHP, or Demerara enriched with fibers and added with minerals;
j. production of vegan artificial honey from the sugars: Coarse, VHP, VVHP, or Demerara enriched with fibers, minerals, and vitamins;
k. artificial honey production from the sugars: Coarse, VHP, VVHP, or Demerara enriched with fibers, minerals, and vitamins and amino acids;
l. artificial honey production from the sugars: Coarse, VHP, VVHP, or Demerara enriched with fibers, minerals, vitamins, amino acids, and encapsulated products; and
m. artificial honey production from beetroot sugar, coconut sugar, and others.

14. PROCESS, as per claim 13, **characterized in that** it produces the artificial honey in items **"a"** through **"m"** covering the following stages:
1 - solid sucrose, present in the carbohydrate source selected in item **"a"** through **"m"** and as described in claims 1 through 3, is diluted with water to a sucrose syrup from 78 to 82 ºBrix and heated at 80 to 85 ºC for total dissolution of the sugar, through agitation in the tank;
2 - When the inversion is performed enzymatically, the syrup is cooled down to 55 ± 1 ºC and the pH adjusted at 4.5 using the acids described in claim 4, and the invertase enzyme described in claims 5 and 6 is added to the syrup;
3 - Optionally, the inversion can be performed chemically, with phosphoric acid, reaching a pH of around 2.0-2.5, the medium with soda ash up to pH 4.5-5.0;
4 - The reaction is kept up to the required rate of inversion of sucrose into glucose and fructose;
5 - The syrup is filtered for removal of particulates through a filter press or a sparkler filter;
6 - In the case of honey production with fibers, especially based on item f of claim 21, the syrup is added with around 0.5 to 20% (w/w) soluble fibers, whether or not organic, such as: corn fibers, polydextroses, soluble maltodextrins, or cassava fibers;
7 - Optionally, the filtered syrup may be added with: (i) aroma identical to the natural flavor, (ii) artificial honey aroma, and/or (iii) optionally, natural honey aroma, in an aroma proportion of 0.2% to 5.0% (w/w) or until it becomes identical to the natural honey aroma;
8 - Optionally, the syrup is added with vitamins, minerals, antioxidants, fructose, and other compounds that improve the properties of the artificial honey product;
9 - In the case of minerals, the syrup may be added in a range from 1.0 to 100.0% for a daily intake of Zinc, Calcium, Phosphorus, Iron, and Magnesium as indicated for an adult person, in addition to the fact that it may contain other minerals, such as copper, selenium, manganese, and phosphorus;
10 - The syrup may be added with amino acids in a range from 1.0 to 2.0% in weight, such as glutamic acid, aspartic acid, glutamine, histidine, glycine, threonine, alanine, arginine, proline, tyrosine, valine, methionine, cysteine, leucine, phenylalanine, isoleucine, tryptophan, ornithine, and lysine, and others;
11 - Finally, the syrup may be optionally added with encapsulated products and can exhibit several properties, such as pharmacological and nutraceutical properties.

## Amended claims

### Amended claims under Art. 19.1 PCT

1. ARTIFICIAL HONEY COMPOSITION refers to inverted sucrose, derived from a plant source, such as sugarcane or beetroot, with conventional or organic certification, **characterized in that** the honey product is produced either chemically or enzymatically, from a syrup with a higher degree of sucrose conversion, between 90% and 98% inversion, exhibiting an equimolar quantity of glucose and fructose close to those of natural bee honey, without using any material or activity of animal origin, with a concentration from 80 to 82° Brix, further exhibiting pharmacological, nutraceutical, and cosmetic properties present in some types of sucrose, having at least one water concentration between 10.0 to 25% (w/w); Fructose at 25.0 to 49.0% (w/w); Glucose at 25.0 to 49.0% (w/w); sucrose at 2 to 40.0% (w/w); 0.1% to 5.0% (w/w) of natural, artificial, or identical honey aroma; total minerals at 0.1 to 2.0% (w/w); total fibers up to 20% (w/w); total vitamins up to 0.1% (w/w), total amino acids up to 2.0% (w/w), natural extracts at 1 to 10.0% (w/w), natural colorants at 1 to 10.0% (w/w), and encapsulated products (w/w), in every 100 g of the end product.

2. COMPOSITION, as per claim 1, sucrose is **characterized in that** it is preferably a sugarcane sugar, chosen from brown sugar, VHP sugar, VVHP sugar, demerara sugar, coarse sugar, or also beetroot sugar, coconut sugar, and others.

3. COMPOSITION, as per claim 1, is **characterized in that** the sucrose inversion, when chemically performed, preferably uses citric acid, phosphoric acid, or even acids allowed on the Positive List, the latter case enabling the production of an organic artificial honey product.

4. COMPOSITION, according to claim 1, is **characterized in that** it contains added fructose, minerals, vitamins, fibers, natural colorants, natural aromas and extracts, and encapsulated substances for the purpose of making it as similar as possible to natural bee honey and adding improved nutraceutical, pharmacological, and organoleptic functions and properties.

5. COMPOSITION, as per claims 1 to 3, is **characterized in that** it has specific final concentrations of 17 to 21% of water (w/w); 35 to 39% of fructose (w/w); 35 to 41% of glucose (w/w); 1.0 to 2.0% of sucrose (w/w); 0.1% to 0.15% (w/w) of natural, artificial, or identical honey aroma; with the optional addition of 0.2 to 2.0% of total minerals (w/w); and 0 to 5.0% of total fibers (w/w), total vitamins at 0 to 0.1% (w/w), total amino acids at 0 to 1.0% (w/w), and encapsulated products at 0 to 10.0 % in every 100 g of the end product.

6. COMPOSITION, as per claims 1 and 4, is **characterized in that** its minerals may contain the following elements in every 100 g of artificial honey: up to 17 mg of sodium; up to 7.0 mg of zinc; up to 3500 mg of potassium; up to 260 mg of magnesium; up to 1000 mg of calcium, and up to 700 mg of iron.

7. COMPOSITION, as per claim 1, **is characterized in that** the addition of the complex-B vitamin is preferably between 0.01 and 0.32 mg in every 100 g of the artificial honey product, so that the composition for final daily intake (mg/100g/day) is inside the following parameters: up to 1.2 mg of Vitamin B1; up to 1.3 mg of vitamin B2; up to 16 mg of vitamin B3; up to 1.3 mg of vitamin B6; up to 0.4 mg of vitamin B9. Moreover, the addition of other vitamins may be: up to 45 mg of vitamin C; up to 0.005 mg of vitamin D; up to 10 mg of vitamin E.

8. COMPOSITION, as per claim 1, **is characterized in that** the fibers are added that can be soluble and insoluble fibers, inserted in the composition either individually or jointly, producing a final food with a low glycemic index and healthier; it is produced from inverted Demerara sugar (>98%), largely converted into glucose and fructose, with a sweetness index very close to bee honey; with added fructose or functional fibers, it exhibits a low glycemic index (<55);

9. COMPOSITION, as per claim 1, **is characterized in that** its pharmacological properties may encompass antioxidant, anti-inflammatory, anti-tumor, and antibacterial activity, depending on the sucrose used in the process.

10. COMPOSITION, as per claims 1 and 3, **characterized in that** its pharmacological activities can be performed by adding the following substances derived from the sugars chosen:
a. Flavonoids, such as luteolin, apigenin, tricine, quercetin, kaempferol;
b. Phenolic, such as caffeic acid, apigenin, luteolin, tricine, chlorogenic acid, coumaric acid, ferulic acid.

11. ARTIFICIAL HONEY PRODUCTION PROCESS, to obtain the composition in the above claims, **characterized in that** it chemically or enzymatically inverts at different inversion rates (sucrose/glucose+fructose ratio from 90% to 98% inversion); contains aromatizers with a honey aroma (artificial, identical to the natural flavor, and optionally a natural aroma); furthermore, it encompasses a honey syrup added with 0.5% to 20.0% (w/w) soluble fibers, whether or not organic, such as corn fibers, polydextroses, soluble maltodextrins. or cassava fibers; a honey syrup is added with 0.5% to 20.0% (w/w) insoluble fibers, whether or not organic, such as corn or cassava fibers or plant fibers; the fact that it is added at 1.0% to 20,0% (w/w) with soluble or insoluble fibers in a proportion of 1.0 to 99.0% (w/w) of mixture of both types of fibers; the syrup may receive additives taking into account the range for a daily intake of Zinc, Calcium, Phosphorus, Iron, and Magnesium.

12. ARTIFICIAL HONEY PRODUCTION PROCESS, as per claim 11, **characterized in that** it can be added with encapsulated, micro-encapsulated, or nano-encapsulated substances, such as sugarcane molasse, sugarcane honey, pharmacological and nutraceutical compounds, natural plant extracts, but not limited to these; the encapsulation process preserves the pharmacological properties of products and masks their potential interferences in the organoleptic properties of artificial honey, however, it maintains their functional properties intact; the addition proportion will depend on the type of product to be added and the concentration of the substances of interest, between 1.0 to 10.0% (w/w) of the end honey product, though it can be higher; some additives like vitamins, amino acids, and minerals may also be encapsulated.

13. ARTIFICIAL HONEY PRODUCTION PROCESS, as per each of the above claims, **characterized in that** it is similar to natural honey from bees, producing an artificial honey composition, whether naturally or not, however without using bees or any other animal, preserving the compounds present in the sucrose used as a raw material, and may exhibit improved functions when compared to bee honey, as well as cover the following types of production:
a. artificial honey production from coarse sugarcane sugar inverted ENZYMATICALLY;
b. artificial honey production from coarse sugarcane sugar inverted CHEMICALLY;
c. artificial honey production from organic coarse sugarcane sugar inverted CHEMICALLY following the Positive List of Organics;
d. artificial honey production from VHP or VVHP sugarcane sugar inverted enzymatically or chemically whether or not following the positive list of organics;
e. artificial honey production from Demerara sugarcane sugar inverted enzymatically or chemically whether or not following the positive list of organics;
f. artificial honey production from the sugars: Coarse, VHP, VVHP, or Demerara enriched with soluble fibers;
g. artificial honey production from the sugars: Coarse, VHP, VVHP, or Demerara enriched with insoluble fibers;
h. artificial honey production from the sugars: Coarse, VHP, VVHP, or Demerara enriched with soluble and insoluble fibers;
i. artificial honey production from the sugars: Coarse, VHP, VVHP, or Demerara enriched with fibers and added with minerals;
j. production of vegan artificial honey from the sugars: Coarse, VHP, VVHP, or Demerara enriched with fibers, minerals, and vitamins;
k. artificial honey production from the sugars: Coarse, VHP, VVHP, or Demerara enriched with fibers, minerals, and vitamins and amino acids;
l. artificial honey production from the sugars: Coarse, VHP, VVHP, or Demerara enriched with fibers, minerals, vitamins, amino acids, and encapsulated products; and artificial honey production from beetroot sugar, coconut sugar, and others.

14. PROCESS, as per claim 13, **characterized in that** it produces the artificial honey in items **"a"** through **"m"** covering the following stages:
1 -solid sucrose, present in the carbohydrate source selected in item **"a"** through **"m"** and as described in claims 1 through 3, is diluted with water to a sucrose syrup from 78 to 82 °Brix and heated at 80 to 85 °C for total dissolution of the sugar, through agitation in the tank;
2 -When the inversion is performed enzymatically, the syrup is cooled down to 55 ± 1 °C and the pH adjusted at 4.5 using the acids described in claim 4, and the invertase enzyme described in claims 5 and 6 is added to the syrup;
3 -Optionally, the inversion can be performed chemically, with phosphoric acid, reaching a pH of around 2.0-2.5, the syrup must be adjusted with calcium carbonate up to pH 4.5-5.0;
4 -The reaction is kept up to the required rate of inversion of sucrose into glucose and fructose;
5 -The syrup is filtered for removal of particulates;
6 -In the case of honey production with fibers, especially based on item f of claim 21, the syrup is added with around 0.5 to 20% (w/w) soluble fibers, whether or not organic, such as: corn fibers, polydextroses, soluble maltodextrins, or cassava fibers;
7 -Optionally, the filtered syrup may be added with: (i) aroma identical to the natural flavor, (ii) artificial honey aroma, and/or (iii) optionally, natural honey aroma, in an aroma proportion of 0.2% to 5.0% (w/w) or preferably with 0.15% aroma identical to the natural flavor;
8 -Optionally, the syrup is added with vitamins, minerals, antioxidants, fructose, and other compounds that improve the properties of the artificial honey product;
9 -In the case of minerals, the syrup may be added in a range from 1.0 to 100.0% (w/w)
for a daily intake of Zinc, Calcium, Phosphorus, Iron, and Magnesium as indicated for an adult person, in addition to the fact that it may contain other minerals, such as copper, selenium, manganese, and phosphorus;
10 -The syrup may be added with amino acids in a range from 1.0 to 2.0% (w/w), such as glutamic acid, aspartic acid, glutamine, histidine, glycine, threonine, alanine, arginine, proline, tyrosine, valine, methionine, cysteine, leucine, phenylalanine, isoleucine, tryptophan, ornithine, and lysine, and others.
11 -Finally, the syrup may be optionally added with encapsulated products and can exhibit several properties, such as pharmacological and nutraceutical properties.
